# FASCICULE DE BREVET EUROPEEN

(11) **EP 3 226 994 B1**
(45) Date de publication et mention de la délivrance du brevet: **07.10.2020**
(21) Numéro de dépôt: 15820217.6
(22) Date de dépôt: 02.12.2015
(51) Int. Cl.: B01D 19/00, B01L 3/00, G01N 7/10, G01N 33/28, H01F 27/08, B65D 51/16, F16B 1/00, H05K 5/02

(54) **VIS MEMBRANAIRE ET PROCEDE D'UTILISATION ASSOCIE**
MEMBRANSCHRAUBE UND VERFAHREN ZUR VERWENDUNG DAVON
MEMBRANE SCREW, AND METHOD FOR USING SAME

(30) Priorité: 03.12.2014 FR 1461824
(43) Date de publication de la demande: 11.10.2017
(73) Titulaire: Electricité de France, 75008 Paris (FR)
(72) Inventeur: ZOUITI, Mohamed, 92320 Chatillon (FR); GUUINIC, Philippe, 75014 Paris (FR)
(74) Mandataire: Plasseraud IP
(86) Numéro de dépôt international: PCT/FR2015/053293
(87) Numéro de publication internationale: WO 2016/087778

(56) Documents cités:
- DE-B3-102010 008 066
- FR-A1- 2 231 963
- JP-A- S57 156 533
- US-A- 4 112 737
- US-A1- 2005 005 674

## Description

La présente invention porte sur une vis membranaire, c'est-à-dire pourvue d'une membrane.

L'invention se situe dans le domaine de l'analyse des propriétés de gaz dissous dans un liquide, tels que par exemple des gaz contenus dans une huile utilisée pour refroidir un transformateur de puissance. US 4 112 737 décrit un appareil relatif à ce domaine.

De manière connue, afin d'analyser l'évolution des propriétés de ce type de liquide, il est nécessaire d'analyser les gaz qu'elle contient.

A cette fin, on a classiquement recours à des dispositifs d'analyse se présentant sous la forme d'un boîtier à connecter fluidiquement à une vanne de transformateur. La vanne est alors ouverte pour autoriser l'admission de l'huile au sein du dispositif et ainsi en rendre possible l'analyse.

Il est également connu de recourir à un dispositif d'analyse connecté à des vannes du transformateur par le biais d'un système de conduites munies d'une pompe de circulation. Sous l'effet de cette pompe, l'huile est acheminée jusqu'au dispositif d'analyse. Une telle configuration présente l'avantage de rendre automatisable l'admission de l'huile dans le dispositif d'analyse.

Toutefois, ces types de dispositifs présentent des inconvénients importants. En effet, dans le premier scénario, la vanne utilisée est rendue indisponible pour une autre opération lorsqu'une analyse doit être menée. En outre, les vannes débouchent généralement sur des zones du transformateur dans lesquelles l'huile à analyser est insuffisamment représentative de l'huile qui est en contact avec les enroulements du transformateur.

Dans le deuxième scénario, ce sont plusieurs vannes qui sont rendues indisponibles le temps de l'opération d'analyse. En outre, la présence du système de conduite complexifie l'installation requise et tend à favoriser les fuites et l'introduction de bulles d'air dans le transformateur.

Aussi, l'invention vient améliorer la situation.

A cet effet, l'invention porte sur une vis membranaire destinée à coopérer avec un orifice d'un conteneur comprenant un liquide et à être au moins partiellement immergée dans le liquide pour séparer des gaz du liquide, dudit liquide, la vis membranaire comprenant :
- un corps de vis comprenant une tête de vis et une portion de fixation pourvue d'un filetage, le corps de vis comprenant un passage central,
- une structure à membrane reçue dans le passage central, la structure à membrane comprenant une membrane pour séparer les gaz du liquide,
la membrane étant agencée en saillie par rapport à la portion de fixation dans une configuration d'utilisation de la vis membranaire prévue pour la mise en contact de la membrane avec le liquide.

Un tel dispositif présente l'avantage de limiter les contraintes associées à la réalisation d'une analyse de liquide, en particulier dans le contexte ci-dessus.

Selon un autre aspect de l'invention, la membrane délimite intérieurement un volume, la vis membranaire comprenant en outre des moyens de modification configurés pour modifier la pression au sein dudit volume, les moyens de modification étant distincts de la membrane.

La présence des moyens de modification permet la génération de dépressions et de surpressions au sein de la membrane, ce qui favorise la pénétration des gaz à travers la membrane dans le cas de dépressions, et favorise l'expulsion des gaz dans le cas de surpression.

Selon un autre aspect de l'invention, les moyens de modifications sont configurés pour modifier la pression au sein du volume en réponse à une variation de la température du liquide.

Ceci permet la génération des dépressions et surpressions de manière simple et fiable.

Selon un autre aspect de l'invention, les moyens de modification comprennent un bloc de matière agencé dans le volume délimité par la membrane ou bien formant une paroi de la structure à membrane.

Les modifications de pression sont ainsi obtenues via un dispositif robuste et peu sujet au vieillissement prématuré.

Selon un autre aspect de l'invention, la vis membranaire comprend en outre une chemise fixée au corps de vis, la membrane étant reçue dans ladite chemise dans la configuration d'utilisation.

La présence de cette chemise permet d'augmenter la tenue mécanique de la vis membranaire et tend à réduire le vieillissement de la membrane en ce qu'elle protège la membrane.

Selon un autre aspect de l'invention, la chemise comprend au moins une perforation pour la mise en contact de la membrane avec le liquide.

Ainsi, la protection conférée par la chemise à la vis n'a que peu voire aucun impact sur l'efficacité de la membrane.

Selon un autre aspect de l'invention, la vis membranaire comprend en outre un bouchon adapté pour être fixé à la tête de vis et maintenir la membrane en place en configuration d'utilisation.

La présence du bouchon tend à limiter les mouvements indésirables de la membrane relativement au reste de la vis et limite ainsi les risques qu'une perte d'efficacité d'analyse ne survienne de ce fait.

Selon un autre aspect de l'invention, la vis membranaire comprend des moyens de sollicitation de la membrane à l'écart de la position de la membrane en configuration d'utilisation.

La présence de ces moyens de sollicitation facilite l'extraction de la membrane de la vis, par exemple pour en réaliser une maintenance ou un remplacement par une nouvelle membrane. Ainsi, on prévient l'occurrence de scénarii dans lesquels la membrane se rompt sous l'effet d'une traction visant à l'extraire de la vis membranaire.

Selon un autre aspect de l'invention, les moyens de sollicitation comprennent un ressort fixé d'une part, à une paroi de la chemise et d'autre part, à un clapet fixé à une extrémité de la membrane, le clapet étant configuré pour obturer le passage central du corps de vis dans une configuration déployée des moyens de sollicitation dans laquelle le ressort est déployé.

Cette configuration des moyens de sollicitation permet d'assurer l'étanchéité de la vis membranaire vis-à-vis du liquide lorsque la membrane est extraite de la vis.

Selon un autre aspect de l'invention, la vis membranaire comprend également un tube engagé dans le corps de vis et faisant saillie hors de la portion de fixation, la membrane étant reçue dans ledit tube.

La présence du tube renforce plus avant la protection de la membrane.

Selon un autre aspect de l'invention, le tube comprend au moins une lumière, le tube étant déplaçable à l'intérieur de la chemise entre une position d'utilisation dans laquelle la lumière est en regard d'une perforation de la chemise pour la mise en contact de la membrane avec le liquide à travers la perforation et la lumière, et une position fermée dans laquelle la lumière est en regard d'une zone de la chemise dépourvue de perforation.

Ceci permet de garantir l'étanchéité de la vis membranaire en cas d'extraction de la membrane.

Selon un autre aspect de l'invention, les moyens de sollicitation comprennent un ressort fixé à la chemise et au tube et configuré pour solliciter le tube vers la position fermée.

Ceci facilite l'obtention d'une disposition étanche de la vis lorsque l'on extrait la membrane dans la mesure où l'opérateur manipulant la vis n'a pas d'action particulière à mener pour éviter que la vis n'autorise le passage du liquide.

L'invention concerne en outre un procédé d'utilisation d'une vis membranaire telle que définie ci-dessus, dans lequel :
- on met en prise la vis membranaire avec les parois d'un orifice d'un conteneur contenant un liquide, et
- on sépare des gaz dissous dans le liquide du liquide via la membrane de la vis membranaire. Selon un autre aspect de l'invention, l'orifice est un orifice de purge d'un radiateur ou d'un boîtier de réfrigération de transformateur de puissance, la membrane étant au contact d'une huile circulant au sein dudit radiateur ou dudit boîtier de réfrigération.

Cette utilisation présente l'avantage d'autoriser de manière très simple l'analyse de l'huile du transformateur via la vis membranaire selon l'invention. En outre, l'huile de transformateur présente une excellente circulation au sein de ce type de dispositifs, de sorte que l'huile qui y circule est parfaitement représentative de l'huile qui passe au contact des enroulements du transformateur. L'analyse qui s'en suit présente ainsi une qualité optimale.

L'invention sera mieux comprise à la lecture de la description détaillée qui va suivre, donnée uniquement à titre d'exemple et faite en référence aux Figures annexées, sur lesquelles :
- les Figures 1a et 1b illustrent une vis membranaire selon l'invention ;
- les Figures 2a et 2b illustrent une vis membranaire selon une première variante de l'invention ;
- les Figures 3a et 3b illustrent une vis membranaire selon une deuxième variante de l'invention ;
- les Figures 4a et 4b illustrent une vis membranaire selon une troisième variante de l'invention ;
- la Figure 5 est un diagramme-bloc illustrant un procédé d'utilisation selon l'invention ; et
- la Figure 6 illustre une étape du procédé de la Figure 5.

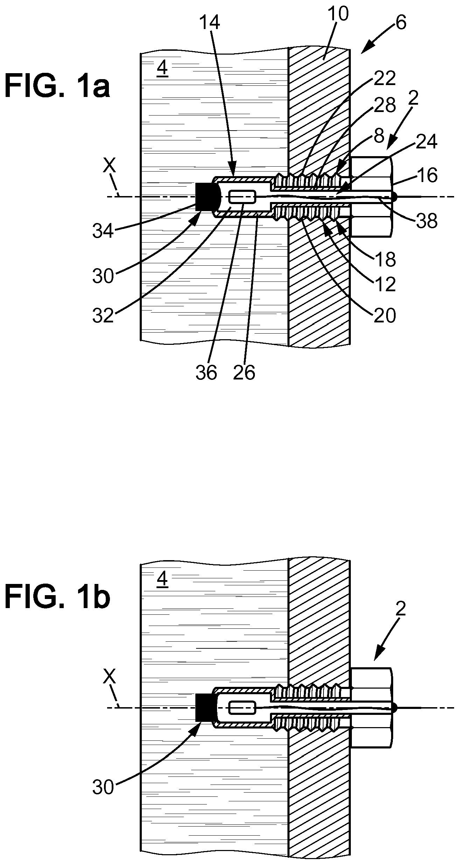

Les Figures 1a et 1b illustrent une vis membranaire 2 selon l'invention, nommée ci-après vis 2.

La vis 2 est adaptée pour être partiellement au contact d'un liquide 4 se trouvant dans un conteneur 6. A cet effet, la vis 2 est prévue pour être engagée dans un orifice 8 d'une enveloppe 10 du conteneur 6 et pour coopérer avec les parois de l'orifice 8 pour son maintien en place. En outre, la vis 2 est adaptée pour faire pénétrer en son sein des gaz initialement dissous dans le liquide 4 en vue de réaliser une analyse physico-chimique de ces gaz.

La vis 2 s'étend sensiblement selon un axe X. La vis 2 comprend un corps de vis 12 et une structure à membrane 14.

Le corps de vis 12 comprend une tête de vis 16 et une portion de fixation 18.

La tête de vis 16 présente une forme connue, par exemple hexagonale. La tête de vis 16 présente des dimensions supérieures aux dimensions de l'orifice 8. La tête de vis 16 est destinée à être en appui contre l'enveloppe 10 une fois la vis 2 engagée dans l'orifice 8.

La portion de fixation 18 présente une forme générale cylindrique s'étendant selon l'axe X. La portion de fixation 18 est pourvue sur sa face externe d'un filetage 20 destiné à coopérer avec un alésage 22 complémentaire ménagé dans les parois délimitant l'orifice 8 au sein de l'enveloppe 10. Cette coopération immobilise la vis 2 en place dans l'orifice 8.

Une fois la vis 2 vissée dans l'orifice 8, la vis 2 étanchéifie l'orifice 8 vis-à-vis du liquide 4, c'est-à-dire que le liquide 4 ne peut pas s'écouler à travers l'orifice 8, et en particulier s'écouler entre la portion de fixation 18 et les parois de l'orifice 8, ou encore entre la tête de vis 16 et l'enveloppe 10.

Le corps de vis 12 comprend en outre un passage central 24 traversant le corps de vis 12 et débouchant au niveau de la tête de vis 16 et de l'extrémité opposée de la portion de fixation 18.

Le corps de vis 12 est par exemple réalisé à partir de métal, ou de tout autre matériau adapté pour être au contact du liquide 4 pendant une période prolongée sans se dégrader.

La structure à membrane 14 est reçue dans le passage central 24 et y est maintenue en place.

La structure à membrane 14 comprend une membrane 26, une manche 28 et des moyens de modifications 30 adaptés pour modifier la pression au sein de la structure à membrane 14.

La membrane 26 est poreuse à tout ou partie des gaz dissous dans le liquide 4 tout en étant imperméable au liquide 4.

Les gaz en question sont par exemple de l'hydrogène, du méthane, de l'éthane, de l'éthylène et de l'acétylène.

A cet effet, la membrane 26 est réalisée à partir d'un matériau perméable au (ou aux) gaz ciblé(s) et imperméable au liquide 4.

La membrane 26 est fixée à l'extrémité de la portion de fixation 18, par exemple par soudage. Parallèlement ou alternativement, la membrane 26 est fixée aux parois du passage central 24 dans la portion de fixation 18 par l'intermédiaire de la manche 28 dont une fonction est cette fixation de la membrane 26 au reste de la vis 2.

La membrane 26 délimite intérieurement un volume 32.

Les moyens de modification 30 sont distincts de la membrane 26. Ils sont prévus pour modifier la pression à l'intérieur du volume 32. En particulier, les moyens de modification 30 sont prévus pour modifier cette pression en réponse à une variation de la température du liquide 4. A cet effet, les moyens de modification 30 comprennent un bloc de matière 34 agencé dans le volume 32 ou bien formant une paroi de la structure à membrane 14.

Ainsi, sous l'effet d'une variation de la température du liquide, le bloc de matière 34 se déforme, modifiant le volume qu'il occupe au sein du volume 32 et donc la pression au sein du volume 32. Le bloc de matière 34 est par exemple réalisé à partir de deux matériaux collés l'un à l'autre et présentant des coefficients de dilatation respectifs différents. Ainsi, la déformation de ces matériaux vient modifier la pression au sein du volume 32.

Dans l'exemple de la Figure la, le bloc 34 forme une extrémité de la paroi de la structure à membrane 14, et est immergé dans le liquide 4. En outre, dans cet exemple, le bloc de matière 34 est dilaté par rapport à sa configuration au repos, générant ainsi une surpression au sein du volume 32. Cette surpression favorise l'expulsion des gaz contenus dans le volume 32. Cette expulsion a par exemple lieu par la tête de la vis 16 à l'aide d'un clapet (non représenté).

Dans l'exemple de la Figure 1b, le bloc 34 est contracté par rapport à sa configuration de repos, générant ainsi une dépression qui favorise la pénétration des gaz dissous dans le liquide 4 par la membrane 26.

Toujours en référence aux Figures la et 1b, la vis 2 comprend également un capteur 36 agencé dans le volume 32. Le capteur 36 est adapté pour analyser les gaz contenus dans le volume 32. Le capteur 36 est raccordé par un câble 38 à un dispositif d'acquisition (non représenté), le câble 38 étant agencé dans la manche 28.

La vis 2 présente une configuration d'utilisation dans laquelle la vis 2 est engagée dans l'orifice 8 et la membrane 26 est en saillie par rapport à la portion de fixation 18 du corps de vis 12. Dans cette configuration, la membrane 26 est au contact du liquide 4. Cette configuration est celle illustrée sur les Figures la et 1b. Par ailleurs, la vis 2 présente une configuration extraite dans laquelle la vis 2 n'est pas engagée dans l'orifice 8 ou la membrane 26 est extraite de la vis 2. Dans l'exemple des Figures la et 1b, dans la configuration extraite (non représentée), la vis 2 n'est pas engagée dans l'orifice 8.

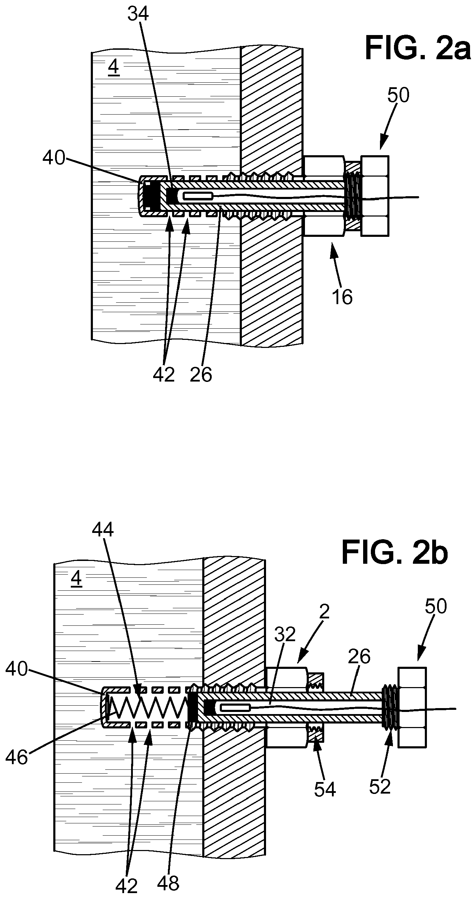

Les Figures 2a et 2b illustrent une variante de la vis 2 selon l'invention.

Dans cette variante, la membrane 26 présente une forme générale cylindrique creuse de diamètre légèrement inférieur à celui du passage central 24. En outre, la membrane 26 n'est pas fixée au corps de vis et peut se déplacer dans le passage central 24. Par ailleurs, le bloc de matière 34 est agencé dans le volume 32.

En outre, la vis 2 comprend une chemise 40 prévue pour recevoir la membrane 26. La chemise 40 est fixée à la portion de fixation 18, et plus particulièrement au niveau de l'extrémité de la portion de fixation 18 opposée à la tête de vis 16. La chemise 40 présente une forme générale cylindrique. En outre, la chemise 40 comprend au moins une perforation 42 pour la mise en contact de la membrane 26 avec le liquide 4.

En outre, dans cette variante, la vis 2 comprend des moyens de sollicitation 44 (Fig. 2b) pour la sollicitation de la membrane 26 à l'écart de la chemise 40 et de la position qu'elle occupe en configuration d'utilisation. En d'autres termes, les moyens de sollicitation 40 sont configurés pour solliciter la vis 2 vers sa configuration extraite. Dans cette configuration extraite, la membrane 26 est positionnée dans le corps de vis 12 et ne fait pas saillie avec la portion de fixation 18.

Ces moyens de sollicitation 44 comprennent un ressort 46 et un clapet 48. Le ressort 46 est fixé d'une part au fond de la chemise 40, et d'autre part au clapet 48. Le clapet 48 est fixé à l'extrémité de la membrane 26.

La vis 2 comprend par ailleurs un bouchon 50 solidaire de la membrane 26. Le bouchon 50 est fixé à l'extrémité de la membrane opposée à la portion de la membrane 26 destinée à être au contact du liquide 4. Le bouchon 50 est adapté pour être fixé de manière libérable à la tête de vis 16. A cet effet, le bouchon 50 présente un moyen de fixation 52, tel que par exemple un filetage, destinée à coopérer avec un moyen de fixation complémentaire porté par la tête de vis 16, tel que par exemple un alésage 54.

En référence à la Figure 2a, en configuration d'utilisation, la membrane 26 est reçue dans la chemise 40. En outre, le ressort 46 est comprimé entre le fond de la chemise 40 et le clapet 48 qui se trouve alors à proximité du fond de la chemise 40. En outre, le liquide 4 est au contact de la membrane 26 à travers les perforations 42. Le bouchon 50 est fixé à la tête de vis, ce qui maintient la membrane 26 en place dans la chemise 40.

En référence à la Figure 2b, en configuration extraite, le bouchon 50 est à l'écart de la tête de vis 16. En outre, la membrane 26 est rétractée dans le passage central 24, le clapet 48 obturant le passage central 24 afin d'étanchéifier la vis 2 vis-à-vis du liquide 4.

Le passage de la configuration extraite de la Figure 2b à la configuration d'utilisation de la Figure 2a s'obtient par déplacement du bouchon 50 à l'encontre de la sollicitation des moyens de sollicitation 44 et vissage du bouchon 50 sur la tête de vis 16. Le passage inverse s'obtient par libération du bouchon 50 par rapport à la tête de vis 16.

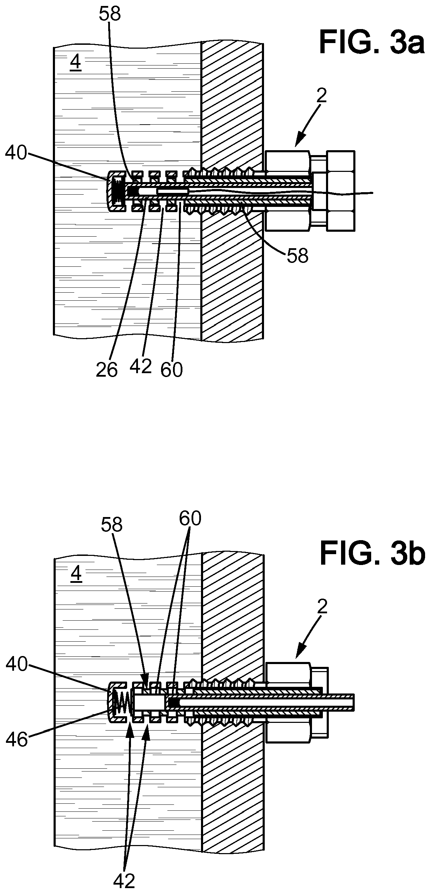

Les Figures 3a et 3b illustrent une variante du mode de réalisation des Figures 2a et 2b.

Dans cette variante, la vis 2 comprend également un tube 58 inséré dans la chemise 40 et destiné à recevoir la membrane 26 en configuration d'utilisation.

Ce tube 58 présente une forme générale cylindrique creuse de diamètre légèrement inférieur à celui de la chemise 40. En outre, son diamètre est légèrement supérieur à celui de la membrane 26.

En outre, le tube 58 présente une ou plusieurs lumières 60 traversantes ménagées dans sa paroi.

Les lumières 60 sont positionnées sur le tube 58 de sorte qu'en configuration d'utilisation, les lumières 60 soient en regard des perforations 42 de la chemise 40. Ceci autorise le passage du liquide 4 à travers les lumières 60 et les perforations 42.

Par ailleurs, dans cette variante, les moyens de sollicitation 44 comprennent simplement le ressort 46, mais pas de clapet. Le ressort est fixé entre le fond de la chemise 40 et le fond du tube 58. Le fond de la chemise 40 est orienté vers le volume 32 et le fond du tube 58 est orienté à l'écart de ce volume 32.

En position déployée du ressort 46, qui correspond à la configuration extraite de la vis 2, les lumières 60 sont en regard d'une zone de la chemise 40 qui est dépourvue de perforation. Ainsi, dans cette configuration, la position relative de la chemise 40 et du tube 58 permet d'étanchéifier la vis 2 du fait que les lumières 60 ne communiquent pas fluidiquement avec les perforations 42.

Les autres aspects de cette variante sont identiques à ceux décrits en référence aux Figures 2a et 2b. En particulier, le passage de la vis 2 entre ses configurations extraite et d'utilisation s'opère de manière identique à celle des Figures 2a et 2b.

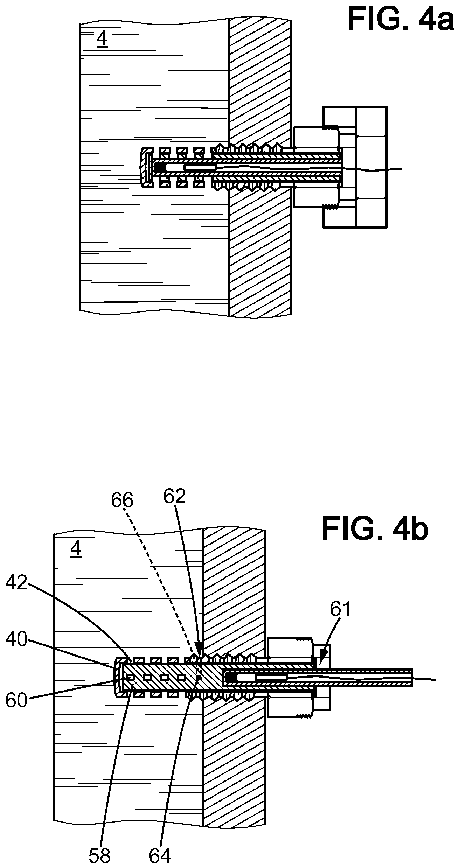

En référence aux Figures 4a et 4b, dans une variante du mode de réalisation des Figures 3a et 3b, la vis 2 est dépourvue de moyens de sollicitation 44.

En outre, le tube 58 est déplaçable au moins en rotation par rapport à la chemise 40 relativement à l'axe X. Plus particulièrement, le tube 58 est pivotable entre une première position correspondant à la configuration d'utilisation et dans laquelle les lumières 60 sont en regard des perforations 42, et une deuxième position correspondant à la configuration extraite dans laquelle les lumières 60 sont en regard d'une zone de la chemise 40 dépourvue de perforations. En outre, le tube 58 est légèrement en saillie relativement à la tête de vis 16. Cette saillie survient par exemple au niveau d'un orifice central 61 de la tête de vis 16 qui est destiné à recevoir une portion complémentaire du bouchon 50. Dans cette variante, le filetage porté par le bouchon 50 est par exemple disposé sur une face interne d'une couronne externe du bouchon 50, l'alésage complémentaire porté par la tête de vis 16 étant agencé sur une face externe de la tête de vis 16.

Avantageusement, le tube 58 et la chemise 40 sont sensiblement immobiles axialement l'un par rapport à l'autre.

A cet effet, la vis 2 comprend des moyens de guidage 62 (Figure 4b) adaptés pour le guidage du tube 58 entre ces première et deuxième positions.

Ces moyens de guidage 62 comprennent par exemple un pion 64 porté par le tube 58 et une gorge 66 portée par la chemise 40 ou par la portion de fixation 18. Le pion 64 est engagé dans la gorge 66. En outre, la gorge 66 est agencée sur une portion circonférentielle de la chemise 40 ou de la portion de fixation 18 et est délimitée par deux butées correspondant respectivement à l'une et l'autre des première et deuxième positions.

Le pivotement du tube 58 à l'intérieur de la chemise 40 est mis en oeuvre par le bouchon 50 lors de sa mise en place et de sa libération vis-à-vis du bouchon 50.

Plus spécifiquement, lors du rapport du bouchon 50 à la tête de vis 16, le pion 64 est en butée contre une extrémité de la gorge 66 et se trouve en deuxième position. Le bouchon 50 entre en contact avec la portion du tube 58 en saillie par rapport à la tête de vis 16. Le vissage du bouchon 50 a pour effet d'entraîner en rotation le tube 58. Le pion 64 est alors guidé dans la gorge jusqu'à la butée opposée de cette dernière, et passe ainsi en première position.

Aussi, préférentiellement, le tube 58 forme une saillie vis-à-vis de la tête de vis 16 de sorte que le bouchon 50 n'entre en contact que lors d'une phase finale de son vissage, le déplacement angulaire du bouchon 50 lors de cette phase finale étant supérieur ou égal au déplacement angulaire du pion 64 requis pour que le pion 64 parcoure l'intégralité de la gorge 66. Ceci limite la compression subie par le tube 58 une fois le bouchon 50 complètement vissé.

A noter qu'alternativement, la gorge 66 est portée par le tube 58 et le pion 64 est porté par la chemise 40. En outre, la gorge 66 couvre par exemple un secteur angulaire d'environ 90°.

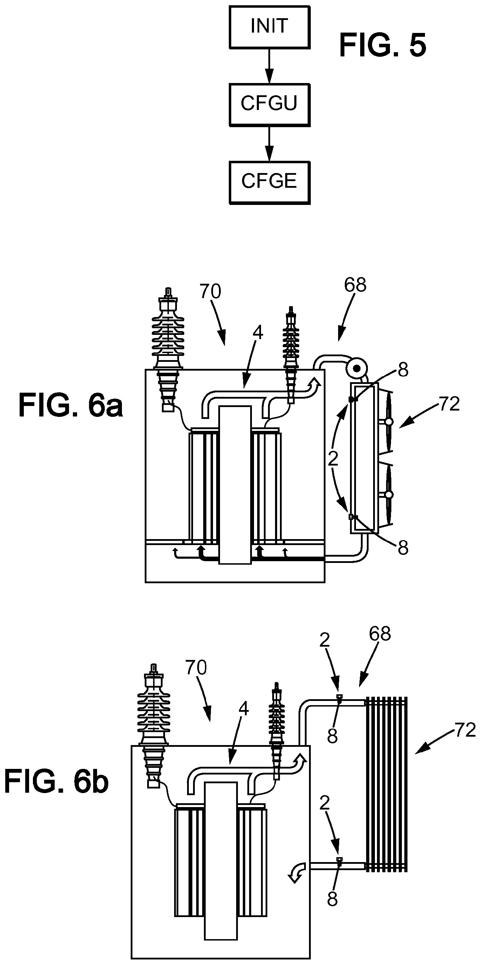

Un procédé d'utilisation de la vis 2 selon l'invention va maintenant être décrit en référence à la Figure 5.

Initialement, lors d'une étape INIT, on engage la vis 2 selon l'invention dans l'orifice 8 de façon à mettre en prise la portion de fixation 18 et les parois délimitant l'orifice 8. Ensuite, on visse la vis 2 jusqu'à obtenir l'étanchéité de l'orifice 8 vis-à-vis du liquide 4.

Si la configuration obtenue à l'issue de ce vissage ne correspond pas à la configuration d'utilisation de la vis 2, on passe la vis en configuration d'utilisation lors d'une étape CFGU. Le détail de ce passage est décrit ci-dessus pour chaque mode de réalisation de l'invention.

Dans cette configuration, comme décrit précédemment, la membrane 26 est au contact du liquide 4. Les gaz dissous dans le liquide 4 pénètrent dans le volume 32 à travers la membrane 26, et sont ensuite analysés par le capteur 36. Celui-ci communique des données représentatives de propriétés des gaz analysés au dispositif d'acquisition via le câble 38.

Ensuite, une fois l'analyse réalisée, lors d'une étape CFGE, on passe la vis 2 en configuration extraite. Cette configuration est par exemple mise en œuvre pour réaliser une maintenance ou remplacer la membrane, ou encore est mise en œuvre lorsqu'une analyse n'est pas nécessaire.

Préférentiellement, en référence aux Figures 6a et 6b, l'orifice 8 est un orifice débouchant dans un système de circulation 68 de liquide de refroidissement d'un transformateur électrique de puissance 70.

Avantageusement, le liquide 4 est de l'huile de refroidissement. En outre, l'orifice 8 est un orifice de purge. Par ailleurs, le système de circulation 68 est un échangeur thermique 72. Plus spécifiquement, l'échangeur thermique 72 est un dispositif aéroréfrigérant (Figure 6a) ou un radiateur (Figure 6b).

L'orifice 8 est par exemple situé sur une conduite d'admission ou de sortie de l'échangeur thermique 72, ou bien directement sur l'échangeur thermique 72.

Une telle utilisation d'une vis 2 selon l'invention présente de nombreux avantageux. En effet, ces orifices de purge sont éloignés de la partie active du transformateur. En outre, cette position au sein d'un échangeur thermique 72 signifie que l'huile vue par la vis 2 est représentative de l'huile qui est au contact des enroulements du transformateur. La qualité de l'analyse menée avec la vis 2 est ainsi optimale.

En outre, une vis 2 ainsi disposée dans un orifice de purge 8 ne rend pas indisponible un autre équipement tel une vanne, et peut tout à fait être utilisée en remplacement d'une vis de purge classique.

D'autres modes de réalisation de l'invention sont également envisageables.

## Revendications

1. Vis membranaire destinée à coopérer avec un orifice (8) d'un conteneur (6) comprenant un liquide (4) et à être au moins partiellement immergée dans le liquide (4) pour séparer des gaz du liquide, dudit liquide, la vis membranaire (2) comprenant :
- un corps de vis (12) comprenant une tête de vis (16) et une portion de fixation (18) pourvue d'un filetage (20), le corps de vis (12) comprenant un passage central (24),
- une structure à membrane (14) reçue dans le passage central (24), la structure à membrane (14) comprenant une membrane (26) pour séparer les gaz du liquide (4),
la membrane (26) étant agencée en saillie par rapport à la portion de fixation (18) dans une configuration d'utilisation de la vis membranaire (2) prévue pour la mise en contact de la membrane (26) avec le liquide (4), la membrane (26) délimitant intérieurement un volume (32), la vis membranaire (2) **caractérisée en ce qu'**elle comprend en outre des moyens de modification (30) configurés pour modifier la pression au sein dudit volume (32), les moyens de modification (30) étant distincts de la membrane (26).

2. Vis selon la revendication 1, **caractérisée en ce que** les moyens de modifications (30) sont configurés pour modifier la pression au sein du volume (32) en réponse à une variation de la température du liquide (4).

3. Vis selon la revendication 1 ou 2, **caractérisée en ce que** les moyens de modification (30) comprennent un bloc de matière (34) agencé dans le volume (32) délimité par la membrane (26) ou bien formant une paroi de la structure à membrane (14).

4. Vis selon l'une quelconque des revendications précédentes, **caractérisé en ce qu'**elle comprend en outre une chemise (40) fixée au corps de vis (12), la membrane (26) étant reçue dans ladite chemise (40) dans la configuration d'utilisation.

5. Vis selon la revendication 4, **caractérisée en ce que** la chemise (40) comprend au moins une perforation (42) pour la mise en contact de la membrane (26) avec le liquide (4).

6. Vis selon l'une quelconque des revendications précédentes, **caractérisée en ce qu'**elle comprend en outre un bouchon (50) adapté pour être fixé à la tête de vis (16) et maintenir la membrane (26) en place en configuration d'utilisation.

7. Vis selon l'une quelconque des revendications précédentes, **caractérisée en ce qu'**elle comprend des moyens de sollicitation (44) de la membrane (26) à l'écart de la position de la membrane en configuration d'utilisation.

8. Vis selon les revendications 4 et 7 prises ensemble, **caractérisée en ce que** les moyens de sollicitation (44) comprennent un ressort (46) fixé d'une part à une paroi de la chemise (40) et d'autre part à un clapet (48) fixé à une extrémité de la membrane (26), le clapet (48) étant configuré pour obturer le passage central (24) du corps de vis (12) dans une configuration déployée des moyens de sollicitation (44) dans laquelle le ressort est déployé.

9. Vis selon l'une quelconque des revendications précédentes, **caractérisée en ce qu'**elle comprend également un tube (58) engagé dans le corps de vis (12) et faisant saillie hors de la portion de fixation, la membrane (26) étant reçue dans ledit tube (58).

10. Vis selon les revendications 4 et 9 prises ensemble, **caractérisé en ce que** le tube (58) comprend au moins une lumière (60), le tube (58) étant déplaçable à l'intérieur de la chemise (40) entre une position d'utilisation dans laquelle la lumière (60) est en regard d'une perforation (42) de la chemise (40) pour la mise en contact de la membrane (26) avec le liquide (4) à travers la perforation (42) et la lumière (60), et une position fermée dans laquelle la lumière (60) est en regard d'une zone de la chemise (40) dépourvue de perforation (42).

11. Vis selon les revendications 7 et 10 prises ensemble, **caractérisée en ce que** les moyens de sollicitation (44) comprennent un ressort (46) fixé à la chemise et au tube et configuré pour solliciter le tube (58) vers la position fermée.

12. Procédé d'utilisation d'une vis membranaire (2) selon l'une quelconque des revendications précédentes, dans lequel :
- on met en prise la vis membranaire (2) avec les parois d'un orifice (8) d'un conteneur (6) contenant un liquide (4), et
- on sépare des gaz dissous dans le liquide (4), dudit liquide, via la membrane (26) de la vis membranaire (2).

13. Procédé d'utilisation selon la revendication 12, dans lequel l'orifice (8) est un orifice de purge d'un radiateur ou d'un boîtier de réfrigération d'un transformateur électrique de puissance (70), la membrane (26) étant au contact d'une huile circulant au sein dudit radiateur ou dudit boîtier de réfrigération.

## Patentansprüche

1. Membranschraube, die dazu bestimmt ist, mit einer Öffnung (8) eines Behälters (6), der eine Flüssigkeit (4) beinhaltet, zusammenzuwirken und mindestens teilweise in die Flüssigkeit (4) einzutauchen, um Gase der Flüssigkeit von der Flüssigkeit zu trennen, wobei die Membranschraube (2) Folgendes beinhaltet:
- einen Schraubenkörper (12), der einen Schraubenkopf (16) und einen Befestigungsabschnitt (18), der ein Gewinde (20) besitzt, beinhaltet, wobei der Schraubenkörper (12) einen zentralen Durchgang (24) beinhaltet,
- eine Membranstruktur (14), die in dem zentralen Durchgang (24) aufgenommen ist, wobei die Membranstruktur (14) eine Membran (26) zum Trennen der Gase der Flüssigkeit (4) beinhaltet,
wobei die Membran (26) in einer Verwendungskonfiguration der Membranschraube (2), die dazu vorgesehen ist, die Membran (26) mit der Flüssigkeit (4) in Kontakt zu bringen, in Bezug auf den Befestigungsabschnitt (18) herausragend angeordnet ist, wobei die Membran (26) innen ein Volumen (32) abgrenzt, wobei die Membranschraube (2) **dadurch gekennzeichnet ist, dass** sie ferner Modifizierungsmittel (30) beinhaltet, die dazu konfiguriert sind, den Druck im Inneren des Volumens (32) zu modifizieren, wobei die Modifizierungsmittel (30) von der Membran (26) verschieden sind.

2. Schraube nach Anspruch 1, **dadurch gekennzeichnet, dass** die Modifizierungsmittel (30) dazu konfiguriert sind, den Druck im Inneren des Volumens (32) als Reaktion auf eine Temperaturänderung der Flüssigkeit (4) zu modifizieren.

3. Schraube nach Anspruch 1 oder 2, **dadurch gekennzeichnet, dass** die Modifizierungsmittel (30) einen Materialblock (34) beinhalten, der in dem durch die Membran (26) abgegrenzten Volumen (32) angeordnet ist bzw. eine Wand der Membranstruktur (14) bildet.

4. Schraube nach einem der vorhergehenden Ansprüche, **dadurch gekennzeichnet, dass** sie ferner einen an dem Schraubenkörper (12) angebrachten Mantel (40) beinhaltet, wobei die Membran (26) in der Verwendungskonfiguration in dem Mantel (40) aufgenommen ist.

5. Schraube nach Anspruch 4, **dadurch gekennzeichnet, dass** der Mantel (40) mindestens eine Perforation (42) beinhaltet, um die Membran (26) mit der Flüssigkeit (4) in Kontakt zu bringen.

6. Schraube nach einem der vorhergehenden Ansprüche, **dadurch gekennzeichnet, dass** sie ferner eine Kappe (50) beinhaltet, die dazu ausgelegt ist, an dem Schraubenkopf (16) angebracht zu sein und die Membran (26) in der Verwendungsposition an ihrem Platz zu halten.

7. Schraube nach einem der vorhergehenden Ansprüche, **dadurch gekennzeichnet, dass** sie Mittel zum Schieben (44) der Membran (26) weg von der Position der Membran in der Verwendungskonfiguration beinhaltet.

8. Schraube nach den Ansprüchen 4 und 7 zusammen, **dadurch gekennzeichnet, dass** die Schiebemittel (44) eine Feder (46) beinhalten, die einerseits an einer Wand des Mantels (40) und andererseits an einer Klappe (48), die an einem Ende der Membran (26) angebracht ist, angebracht ist, wobei die Klappe (48) dazu konfiguriert ist, den zentralen Durchgang (24) des Schraubenkörpers (12) in einer gestreckten Konfiguration der Schiebemittel (44), in der die Feder gestreckt ist, abzudichten.

9. Schraube nach einem der vorhergehenden Ansprüche, **dadurch gekennzeichnet, dass** sie auch ein Rohr (58) beinhaltet, das in den Schraubenkörper (12) eingeführt ist und aus dem Befestigungsabschnitt herausragt, wobei die Membran (26) in dem Rohr (58) aufgenommen ist.

10. Schraube nach den Ansprüchen 4 und 9 zusammen, **dadurch gekennzeichnet, dass** das Rohr (58) mindestens einen Kanal (60) beinhaltet, wobei das Rohr (58) im Inneren des Mantels (40) zwischen einer Verwendungsposition, in der sich der Kanal (60) gegenüber einer Perforation (42) des Mantels (40) befindet, um die Membran (26) durch die Perforation (42) und den Kanal (60) mit der Flüssigkeit (4) in Kontakt zu bringen, und einer geschlossenen Position, in der sich der Kanal (60) gegenüber einem Bereich des Mantels (40), der keine Perforation (42) besitzt, befindet, verschiebbar ist.

11. Schraube nach den Ansprüchen 7 und 10 zusammen, **dadurch gekennzeichnet, dass** die Schiebemittel (44) eine Feder (46) beinhalten, die an dem Mantel und an dem Rohr angebracht ist und dazu konfiguriert ist, das Rohr (58) zu der geschlossenen Position hin zu schieben.

12. Verfahren zur Verwendung einer Membranschraube (2) nach einem der vorhergehenden Ansprüche, wobei:
- die Membranschraube (2) mit den Wänden einer Öffnung (8) eines Behälters (6), der eine Flüssigkeit (4) enthält, in Eingriff gebracht wird und
- in der Flüssigkeit (4) gelöste Gase mittels der Membran (26) der Membranschraube (2) von der Flüssigkeit getrennt werden.

13. Verwendungsverfahren nach Anspruch 12, wobei die Öffnung (8) eine Entlüftungsöffnung eines Heizkörpers oder eines Kühlkastens eines elektrischen Leistungstransformators (70) ist, wobei die Membran (26) mit einem Öl, das im Inneren des Heizkörpers oder des Kühlkastens zirkuliert, in Kontakt ist.

## Claims

1. Membrane screw intended to cooperate with an orifice (8) of a container (6) comprising a liquid (4) and being at least partially immersed in the liquid (4) in order to separate gases of the liquid from said liquid, the membrane screw (2) comprising:
- a screw body (12) comprising a screw head (16) and a fixing portion (18) provided with a thread (20), the screw body (12) comprising a central passage (24),
- a structure with a membrane (14) received in the central passage (24), the structure with a membrane (14) comprising a membrane (26) for separating the gases from the liquid (4),
the membrane (26) being arranged projecting with respect to the fixing portion (18) in a use configuration of the membrane screw (2) provided for putting the membrane (26) in contact with the liquid (4), the membrane (26) internally delimiting a volume (32), the membrane screw (2) **characterised in that** it furthermore comprises modification means (30) configured to modify the pressure in said volume (32), the modification means (30) being distinct from the membrane (26).

2. Screw according to claim 1, **characterised in that** the modification means (30) are configured to modify the pressure in the volume (32) in response to a variation in the temperature of the liquid (4).

3. Screw according to claim 1 or 2, **characterised in that** the modification means (30) comprise a block of material (34) arranged in the volume (32) delimited by the membrane (26) or forming a wall of the structure with a membrane (14).

4. Screw according to any one of the preceding claims, **characterised in that** it further comprises a jacket (40) fixed to the screw body (12), the membrane (26) being received in said jacket (40) in the use configuration.

5. Screw according to claim 4, **characterised in that** the jacket (40) comprises at least one perforation (42) for putting the membrane (26) in contact with the liquid (4).

6. Screw according to any one of the preceding claims, **characterised in that** it further comprises a plug (50) adapted to be fixed to the screw head (16) and to hold the membrane (26) in place in the use configuration.

7. Screw according to any one of the preceding claims, **characterised in that** it comprises means (44) for urging the membrane (26) away from the position of the membrane in the use configuration.

8. Screw according to claims 4 and 7 taken together, **characterised in that** the urging means (44) comprise a spring (46) fixed firstly to a wall of the jacket (40) and secondly to a valve (48) fixed to one end of the membrane (26), the valve (48) being configured to close off the central passage (24) of the screw body (12) in a deployed configuration of the urging means (44) in which the spring is deployed.

9. Screw according to any one of the preceding claims, **characterised in that** it also comprises a tube (58) engaged in the screw body (12) and projecting out of the fixing portion, the membrane (26) being received in said tube (58).

10. Screw according to claims 4 and 9 taken together, **characterised in that** the tube (58) comprises at least one aperture (60), the tube (58) being movable inside the jacket (40) between a use position in which the aperture (60) is opposite a perforation (42) in the jacket (40) for putting the membrane (26) in contact with the liquid (4) through the perforation (42) and the aperture (60), and a closed position in which the aperture (60) is opposite a zone of the jacket (40) with no perforation (42).

11. Screw according to claims 7 and 10 taken together, **characterised in that** the urging means (44) comprise a spring (46) fixed to the jacket and to the tube and configured to urge the tube (58) towards the closed position.

12. Method for using a membrane screw (2) according to any one of the preceding claims, wherein:
- the membrane screw (2) is engaged with the walls of an orifice (8) of a container (6) containing a liquid (4), and
- gases dissolved in the liquid (4) are separated from said liquid, via the membrane (26) of the membrane screw (2).

13. Method of use according to claim 12, wherein the orifice (8) is a drainage orifice of a radiator or of a refrigeration box of a power electrical transformer (70), the membrane (26) being in contact with an oil circulating in said radiator or said refrigeration box.
